# EUROPÄISCHE PATENTSCHRIFT

(11) **EP 1 343 471 B1**
(45) Veröffentlichungstag und Bekanntmachung des Hinweises auf die Patenterteilung: **22.11.2006**
(21) Anmeldenummer: 01985802.6
(22) Anmeldetag: 20.12.2001
(51) Int. Cl.: A61K 9/00

(54) **MIKROSYSTEM ZUR KONTROLLE DER WIRKSTOFFABGABE AUS EINEM WIRKSTOFFRESERVOIR**
MICROSYSTEM FOR CONTROLLED DISPENSATION OF AN ACTIVE SUBSTANCE FROM A RESERVOIR
MICROSYSTEME SERVANT A CONTROLER LA DISTRIBUTION D'UN PRINCIPE ACTIF CONTENU DANS UN RESERVOIR

(30) Priorität: 20.12.2000 DE 10063612
(43) Veröffentlichungstag der Anmeldung: 17.09.2003
(73) Patentinhaber: Fraunhofer-Gesellschaft zur Förderung der angewandten Forschung e.V., 80686 München (DE)
(72) Erfinder: THIELECKE, Hagen, 66440 Blieskastel (DE); ROBITZKI, Andrea, 68519 Viernheim (DE)
(74) Vertreter: Gagel, Roland
(86) Internationale Anmeldenummer: PCT/DE2001/004874
(87) Internationale Veröffentlichungsnummer: WO 2002/056862

(56) Entgegenhaltungen:
- WO-A-95/30892
- WO-A-98/00107
- US-A- 4 548 955

## Beschreibung

### Technisches Anwendungsgebiet

Die vorliegende Erfindung betrifft ein Mikrosystem zur Kontrolle der Wirkstoffabgabe bzw. -freisetzung aus einem Wirkstoffreservoir, mit einem dünnen Trägersubstrat, das aus einem für den Wirkstoff undurchlässigen Material besteht und eine oder mehrere Durchgangsöffnungen für den Wirkstoff aufweist, mehreren im Bereich der Durchgangsöffnungen angeordneten Elektroden sowie einer in das Trägersubstrat integrierten Elektronik zur Ansteuerung der Elektroden. Die Erfindung betrifft weiterhin ein medizinisches Implantat mit einem derartigen Mikrosystem.

Die kontrollierte bzw. gesteuerte lokale in vivo Freisetzung von Wirkstoffen im Körper eines Patienten hat große Bedeutung bei der Therapie von Krankheiten sowie zur Verringerung von Nebenwirkungen beim Einsatz von Implantaten. Medizinische Implantate verursachen häufig unerwünschte Reaktionen wie beispielsweise Infektionen im menschlichen Körper, die durch eine Schädigung des Epithliums, durch eine Begünstigung des Mikroorganismenwachstums, durch eine Beeinflussung der Immunabwehr oder durch kontaminierte Implantate hervorgerufen werden können. Das Risiko unerwünschter Reaktionen des Körpers kann durch den Einbau von miniaturisierten Vorrichtungen in die Implantate minimiert werden, die beispielsweise geeignete Wirkstoffe zur Vermeidung von Infektionen freisetzen. Eine weiterer Bedarf für eine kontrollierte lokale Freisetzung von Medikamenten besteht bei der Therapie bestimmter Krankheiten, beispielsweise zur Tumorbekämpfung.
Mikrosysteme, wie das der vorliegenden Erfindung, eignen sich aufgrund ihrer geringen Abmessungen für den Einsatz zur lokalen und kontrollierten Wirkstofffreisetzung im menschlichen Körper in besonderer Weise.

### Stand der Technik

Die kontrollierte Wirkstofffreisetzung im menschlichen Körper über einen bestimmten Zeitbereich wird derzeit mit unterschiedlichen Systemen realisiert. So ist es bekannt, die Wirkstoffe in ein Polymer einzubetten, aus dem sie in einer bekannten Zeitspanne ausdiffundieren. Weiterhin können Polymere eingesetzt werden, die durch eine zeitlich vorbestimmte Degradation im Körper die Wirkstoffe kontrolliert freisetzen. Mit Hilfe dieser Systeme kann die Wirkstoffkonzentration mit nur einer Dosis im gewünschten therapeutischen Bereich gehalten werden. Die Systeme ermöglichen bei Implantation die Begrenzung der Wirkstofffreisetzung auf bestimmte Körperteile, so dass die erforderliche Menge an systemischen Medikamenten reduziert und die Zersetzung von größeren Wirkstoffmengen durch den Körper verhindert wird.
Einen Überblick über bekannte Systeme zur kontrollierten Wirkstofffreisetzung gibt beispielsweise der Artikel von J.M. Schierholz et al., "Sophisticated Medical Devices as Local Drug-Delivery Systems", Med. Device Technol. 11, 12-17 (2000).

Es gibt jedoch eine Vielzahl von klinischen Situationen, für die die Freisetzung einer zeitlich konstanten Rate von Wirkstoffen nicht ausreicht. Als Beispiele hierfür können die Freisetzung von Insulin bei Patienten mit Diabetes mellitus, von Antiarrythmie-Therapeutika für Patienten mit Herzrhythmusstörungen, von Magensäure-Inhibitoren zur Geschwürbehandlung, von Nitriden für Patienten mit Angina Pectoris sowie der Einsatz zur selektiven β-Rezeptorblockade, zur Geburtskontrolle, zum Hormonersatz, zur Immunisierung und zur Krebstherapie angeführt werden. In derartigen Anwendungsfällen muss der zeitliche Ablauf der Wirkstofffreisetzung gezielt auf die physiologischen Erfordernisse abstimmbar sein.
Für eine bessere Kontrolle der Wirkstofffreisetzung, insbesondere zur Realisierung einer aktiv steuerbaren Abgabe, wurden Polymere entwickelt, die sich durch äußere Einwirkungen, wie Ultraschall, elektrische oder magnetische Felder, Licht, Enzyme, pH-Wert- und Temperaturänderungen zur gesteuerten Freisetzung der darin eingebetteten Wirkstoffe ansteuern lassen. Einen Überblick über derartige Systeme gibt beispielsweise der Artikel von J. Kost et al., "Responsive Polymer Systems for Controlled Delivery of Therapeutics", Trends Biotechnol. 10, 127 - 131 (1992).

Ein weit verbreiteter Nachteil dieser Polymerbasierenden Systeme liegt jedoch in ihrer zum Teil mangelnden Biokompatibilität und unerwünschten Toxizität, der relativ langen Reaktionszeit auf Stimuli, der begrenzten Lebensdauer sowie einer in vielen Fällen nicht ausreichenden Reproduzierbarkeit der Wirkstoffabgabe. Weiterhin wird die Lieferung praxisrelevanter Wirkstoffmengen aufgrund der begrenzten Aufnahmefähigkeit der Polymere erschwert.

In der US 4,003,779, der US 4,146,029, der US 3,692,027 sowie der US 4,360,019 sind Mikrosysteme zur kontrollierten Wirkstofffreisetzung beschrieben, die mechanische Mechanismen, insbesondere miniaturisierte, elektrisch angetriebene Komponenten, zur Wirkstoffabgabe aus einem Reservoir einsetzen. Gerade die Zuverlässigkeit der Wirkstofffreisetzung bei kleinen Abgabemengen bereitet bei derartigen mechanischen Systemen jedoch noch Probleme.

Ein weiterer Mechanismus zur kontrollierten Freisetzung von Wirkstoffen aus einem Wirkstoffreservoir wird in der US 5,556,528 beschrieben. Diese Druckschrift setzt als Mikrosystem eine nanoporöse Membran ein, beispielsweise aus P2VB, auf der eine aktive Kontrollstruktur in Form einer dünnen Schicht aus Molekülen mit einem Dipolmoment von größer als 5 Debye aufgebracht ist. Diese dünne Kontrollschicht hat die Eigenschaft, dass sie ihre Permeabilität für die freizusetzenden Wirkstoffe in Abhängigkeit von der einwirkenden elektrischen Feldstärke ändert. Dieser als Elektroporation aus dem Bereich der Zellforschung bekannte Effekt ermöglicht, die Kontrollschicht über eine elektrische Ansteuerung für die Wirkstoffe permeabel oder impermeabel zu machen. Das zugrunde liegende Prinzip ist beispielsweise der Veröffentlichung von P.F. Lurquin, "Gene Transfer by Electroporation", Mol. Biotechnol. 7, 5-35 (1997) zu entnehmen.

Eine konkrete Realisierung eines funktionsfähigen Mikrosystems für den Einsatz im menschlichen Körper auf Basis des Prinzips der Elektroporation ist der US 5,556,528 jedoch nicht zu entnehmen.

Die US 5,797,898 beschreibt ein Mikrosystem aus einem planaren Mikrochip, in den Kavitäten als Wirkstoffreservoirs geätzt sind. Die Wirkstoffreservoirs sind mit Reservoirkappen verschlossen, die aus elektrisch leitfähigen Schichten bestehen und sich bei Beaufschlagung mit einem elektrischen Potential elektrochemisch zersetzen. Der als Ausführungsbeispiel offenbarte Mikrochip weist eine Dicke von 300 µm auf und beinhaltet mehrere arrayförmig angeordnete Wirkstoffreservoirs mit Öffnungsflächen von 30 x 30 µm. In den Mikrochip ist eine Elektronik für die Ansteuerung der als Elektroden ausgebildeten elektrisch leitfähigen Schichten zum Verschluss der Mikroreservoirs integriert. Ein derartiges Mikrosystem lässt sich mit bekannten Techniken der Halbleitertechnologie realisieren.
Durch Ansteuerung der entsprechenden Elektroden lassen sich die elektrisch leitfähigen Reservoirkappen gezielt zersetzen, so dass der darunter im Wirkstoffreservoir befindliche Wirkstoff freigesetzt wird. Eine wiederholte gesteuerte Wirkstofffreisetzung aus einem einzelnen Reservoir ist mit dieser Technik jedoch nicht möglich. Hierfür muss eine Vielzahl von einzelnen Reservoirs vorgesehen werden, die entsprechend der gewünschten Wirkstofffreisetzung nacheinander geöffnet werden können. Ein weiterer Nachteil dieses Mikrosystems besteht darin, dass das Material der Reservoirkappen bei der Zersetzung in die physiologische Umgebung abgegeben wird. Aufgrund der Vielzahl von Reservoirs, die für eine wiederholte gesteuerte Wirkstoffabgabe notwendig sind sowie der relativ starren Struktur dieses Mikrosystems stößt dessen Einsatz bei Anwendungen mit kritischen Platzverhältnissen, beispielsweise in Verbindung mit kleinen Prothesen oder in bestimmten Körperregionen, wie dem Gehirn, auf Probleme.

Die Aufgabe der vorliegenden Erfindung besteht darin, ein Mikrosystem sowie die Ausgestaltung eines Implantates mit einem Mikrosystem anzugeben, die eine ereignisgesteuerte in vivo Freisetzung von Wirkstoffen auch bei Anwendungen mit sehr kritischen Platzverhältnissen ermöglichen.

### Darstellung der Erfindung

Die Aufgabe wird mit dem Mikrosystem sowie dem Implantat gemäß den Patentansprüchen 1 bzw. 21 gelöst. Vorteilhafte Ausgestaltungen des Mikrosystems sowie des Implantates sind Gegenstand der Unteransprüche.

Das vorliegende Mikrosystem besteht aus einem dünnen, mechanisch flexiblen Trägersubstrat, das aus einem für den Wirkstoff undurchlässigen Material besteht und eine oder mehrere Durchgangsöffnungen für den Wirkstoff aufweist. Die Durchgangsöffnungen sind als Mikrokanäle und/oder Mikrospalte ausgebildet und auf einer Seite des Trägersubstrates mit einer Schicht aus einem elektroporösen Material überdeckt. Beidseitig der Mikrokanäle und/oder Mikrospalte sind Elektroden ausgebildet, die über eine in das Trägersubstrat integrierte Elektronik ansteuerbar sind.

Das elektroporöse Material besteht vorzugsweise aus einem Lipidfilm, in dem sich durch Anlegen eines elektrischen Feldes reversibel Poren erzeugen lassen, die für die freizusetzenden Wirkstoffe permeabel sind. Die Mikrokanäle und/oder Mikrospalte sind derart dimensioniert, dass sie einerseits den Durchgang der Wirkstoffe ermöglichen und andererseits eine Überdeckung mit einem zusammenhängenden Lipidfilm zulassen. Vorzugsweise weisen diese Mikrospalte bzw. Mikrokanäle dafür eine Öffnungsbreite von maximal 10 µm auf.

Das Trägersubstrat besteht vorzugsweise aus einem gedünnten Silizium-Substrat, in das die Mikrospalte und/oder Mikrokanäle eingeätzt wurden. Für eine ausreichende Flexibilität dieses Trägersubstrates ist es erforderlich, dass dessen Dicke geringer als etwa 80 µm ausfällt. Die Elektroden werden hierbei vorzugsweise als dünne Elektrodenbereiche auf das Trägersubstrat aufgebracht oder in dessen Oberfläche integriert, wobei die Schicht aus elektroporösem Material auf diese Elektroden bzw. Bereiche hiervon aufgebracht wird. Ein derartiger Aufbau lässt sich mit bekannten Techniken der Halbleitertechnologie auf einfache Weise realisieren.

Das vorliegende Mikrosystem, im Folgenden aufgrund seiner geringen Dicke und großen Flexibilität auch als Foliensystem bezeichnet, wird so auf Wirkstoffreservoirs, die beispielsweise in Prothesen eingearbeitet sein können, aufgebracht, dass die Folienstruktur die Wirkstoffe gegen die physiologische Umgebung abdichtet. Neben den in den Implantaten integrierten Wirkstoffdepots lassen sich derartige Depots selbstverständlich auch als feste Schicht auf das Foliensystem aufbringen.
Durch die Elektroden mit der in dem System integrierten Elektronik lässt sich über der Schicht aus elektroporösem Material ein elektrisches Feld anlegen, durch das in den Bereichen dieser Schicht, die sich über den Mikrospalten befinden, reversibel Poren erzeugt werden. In Abhängigkeit vom zeitlichen Verlauf der Feldstärke und der Art des elektroporösen Materials existieren die Poren entweder zeitlich begrenzt oder permanent. Solange die Poren ausgebildet sind, werden Wirkstoffe aus den Depots durch die Poren hindurch in die physiologische Umgebung abgegeben. Werden die elektrischen Felder abgeschaltet, so schließen sich die Poren und die Wirkstoffabgabe wird unterbrochen.
Die Steuerung der an den Elektroden anliegenden Spannung über die Elektronik kann durch unterschiedliche Komponenten getriggert werden. In einer Ausführungsform kann hierfür ein Biosensor eingesetzt werden, der das Triggersignal in Abhängigkeit von der Konzentration bestimmter Wirkstoffe in der Umgebung erzeugt. Weiterhin lassen sich die Steuersignale über eine Telemetrie oder durch eine entsprechende Software mit vorgegebenem Programmablauf, die in einem auf dem Trägersubstrat integrierten Programmspeicher vorgesehen sein kann, an die Elektronik übermitteln. Der Biosensor sowie Anschlüsse für Triggersignale können direkt auf dem Mikrosystem angebracht oder in dieses integriert sein.
Die Spannungsversorgung der Elektronik kann beispielsweise drahtlos über ein induktives Verfahren mit einer körperexternen Sendespule erfolgen. Die Empfängerspule kann in das Foliensystem integriert oder separat ausgeführt sein. Um eine kontinuierliche Energieversorgung zu sichern und um die notwendige Energie für die Erzeugung der elektrischen Felder über die elektroporöse Schicht bereitzustellen, kann auch ein Energiespeicher auf dem Foliensystem vorgesehen sein. Dieser Energiespeicher lässt sich beispielsweise über entsprechende Spulen kontinuierlich von außen induktiv laden, so dass er bei kurzzeitigem Energiebedarf die volle Leistung liefern kann.

Aufgrund seiner flexiblen Ausgestaltung kann das vorliegende Mikrosystem flexibel an die Form von Prothesen sowie die physiologischen Platzverhältnisse im Körper angepasst werden, so dass es nur minimalen Raum erfordert. Es lässt sich leicht mit Prothesen kombinieren, ohne dass deren Funktion beeinträchtigt wird. Selbstverständlich lässt sich das Mikrosystem mit entsprechend daran angebrachten Wirkstoffreservoirs auch separat, beispielsweise in Blutbahnen, einsetzen. Durch seine flexible Ausgestaltung kann es hierbei optimal an die jeweilige Umgebung angepasst werden.
Das Mikrosystem eignet sich besonders vorteilhaft für Anwendungen in Verbindung mit kleinen Prothesen, wie beispielsweise Stents, die in Gefäße wie die Blutbahn, Herzarterien oder die Luftröhre eingesetzt werden. Gerade bei derartigen Anwendungen bereiten die bekannten Systeme des Standes der Technik aufgrund ihres nicht zu vernachlässigenden Platzbedarfs Probleme.
Die Elektroden können durch eine Elektronik angesteuert werden, die durch physiologische Ereignisse getriggert wird. Daher kann eine Wirkstofffreisetzung in Abhängigkeit von den physiologischen Erfordernissen erfolgen. Eine Fehlauslösung aufgrund externer elektrischer oder magnetischer Felder kann ausgeschlossen werden. Durch eine reversible Ausgestaltung der Porenbildung in der elektroporösen Schicht, insbesondere einer Lipidmembran, kann die gesteuerte Wirkstoffabgabe aus einem einzelnen Depot wiederholt erfolgen. Die Materialien, die die Wirkstoffreservoirs beim vorliegenden Mikrosystem abdecken, gehen bei der Wirkstofffreisetzung nicht in die physiologische Umgebung über.

In einer besonders vorteilhaften Ausführungsform befindet sich die Schicht aus elektroporösem Material zwischen zwei Substraten mit entsprechenden Mikrospalten bzw. Mikrokanälen, deren Öffnungen übereinander liegen, so dass die Mikrospalte bzw. Mikrokanäle des einen Substrates mit den Mikrospalten bzw. Mikrokanälen des anderen Substrates jeweils eine einzigen Durchflusskanal bilden, dessen Durchfluss über die Schicht aus elektroporösem Material gesteuert werden kann. Diese Anordnung hat den Vorteil eines besseren Schutzes der Schicht aus elektroporösem Material. Weiterhin kann eine derartige Anordnung auf einfachere Weise, d.h. ohne Rücksicht auf die Schicht aus elektroporösem Material, an einer Prothese befestigt werden. Die Elektroden können in gleicher Weise wie bei einem einfachen Trägersubstrat auf das Substrat aufgebracht oder in dessen Oberfläche integriert sein. Hierbei können Elektroden in beiden Substraten vorgesehen sein oder auch nur in einem der beiden Substrate. Auch eine wechselseitige Anordnung, d.h. eine Elektrode auf einer Seite des Mikrospaltes in einem Substrat und die andere Elektrode auf der anderen Seite des Mikrospaltes im anderen Substrat, ist denkbar. Für die Anordnung der Elektroden ist es bei der vorliegenden Erfindung lediglich erforderlich, dass über diese Elektroden das elektroporöse Material im Bereich der Mikrokanäle bzw. Mikrospalte mit einem ausreichend hohen elektrischen Feld beaufschlagbar ist, um durch eine Porenbildung eine Wirkstofffreisetzung ermöglichen zu können. Die genaue Anordnung der Elektroden zur Erzielung dieser Wirkung ist für die vorliegende Erfindung nicht wesentlich und bleibt dem Fachmann überlassen.
Eine derartige Anordnung aus einer Schicht aus elektroporösem Material zwischen zwei Trägersubstraten lässt sich sehr vorteilhaft dadurch realisieren, dass ein entsprechend größeres Substrat mit der doppelten Fläche der Einzelsubstrate hergestellt, zur Erzeugung der Mikrokanäle, Elektroden, Elektronik und elektroporösen Schicht bearbeitet und anschließend zusammen gefaltet wird.

Das Trägersubstrat muss nicht unbedingt aus einem Siliziumchip bestehen. Es kann beispielsweise auch aus einem Polyimidfilm gebildet werden, in den ein dünner Siliziumchip hybrid integriert wird.
Zur Erhöhung der Verträglichkeit des Trägersubstrates mit der physiologischen Umgebung kann dieses auf der mit dieser Umgebung in Kontakt kommenden Oberfläche mit einem biokompatiblen Material beschichtet werden.
In einer Weiterbildung des Mikrosystems werden die Oberfläche des Trägersubstrates, die der elektoporösen Schicht gegenüber liegt und mit der physiologischen Umgebung in Kontakt kommt, sowie die Innenwände der Durchgangsöffnungen in besonderer Weise ausgestaltet. Die Oberfläche der Öffnungsbereiche und der Innenwände der Öffnungen ist dabei so gestaltet, dass eine Zelladhäsion an der Oberfläche vermieden wird. Die Oberfläche zwischen den Öffnungen ist so gestaltet, dass Zellen adhärieren können. Dadurch wird einerseits ein Einwachsen von Zellen in die Öffnungen und so eine Schädigung der Lipidmembran vermieden und anderseits eine Fixierung des Systems erreicht. Die Oberflächenmodifizierung kann durch Nanostrukturierung und/oder durch Beschichtung mit einem geeignetem Material erfolgen.

Das vorliegende Mikrosystem lässt sich in sehr vorteilhafter Weise in Kombination mit einem Implantat einsetzen, in das Wirkstoffreservoirs eingearbeitet sind. Das Mikrosystem wird dabei derart an dem Implantat befestigt, insbesondere angeklebt, dass die Mikrospalte und/oder Mikrokanäle jeweils über Wirkstoffreservoirs liegen. Weiterhin werden die einzelnen Bereiche zwischen den Wirkstoffreservoirs zusätzlich durch das Mikrosystem abgedichtet. Durch Integration der Wirkstoffreservoirs in das Implantat lässt sich auf diese Weise eine sehr platzsparende Anordnung zur Wirkstofffreisetzung realisieren, da sich das vorliegende Mikrosystem flexibel an die Oberfläche des Implantates anpasst und nur eine sehr geringe Dicke aufweist.
Selbstverständlich lassen sich die Wirkstoffreservoirs auch beispielsweise als Festphase auf dem Implantat fixieren, auf die dann das vorliegende Mikrosystem aufgebracht wird.

Das vorliegende Mikrosystem sowie das Implantat mit einem derartigen Mikrosystem werden nachfolgend anhand von Ausführungsbeispielen in Verbindung mit den Figuren ohne Beschränkung des allgemeinen Erfindungsgedankens nochmals kurz erläutert. Hierbei zeigen:
- Fig. 1: eine schematische Schnittdarstellung eines flexiblen Mikrosystems an einer Prothese gemäß einem Ausführungsbeispiel der vorliegenden Erfindung;
- Fig. 2: ein Beispiel für die im Mikrosystem realisierte Elektrodensteuerung im Blockschaltbild;
- Fig. 3: ein Beispiel für das Mikrosystem zur kontrollierten Wirkstofffreisetzung in Schnittdarstellung (a) sowie im Layout (b);
- Fig. 4: ein Beispiel für das Mikrosystem in Kombination mit Wirkstoffreservoirs;
- Fig. 5: ein Beispiel für ein Mikrosystem gemäß einer weiteren Ausgestaltung der vorliegenden Erfindung mit eingeschlossenem Lipidfilm in schematischer Schnittdarstellung (a) sowie im Elektroden-Layout (b);
- Fig. 6: ein weiteres Beispiel für ein Mikrosystem gemäß der vorliegenden Erfindung mit eingeschlossenem Lipidfilm in schematischer Schnittdarstellung (a) sowie im Elektroden-Layout (b); und
- Fig. 7: zwei Beispiele für ein erfindungsgemäßes Mikrosystem mit eingebetteter Lipidschicht in Kombination mit Wirkstoffdepots.

### Wege zur Ausführung der Erfindung

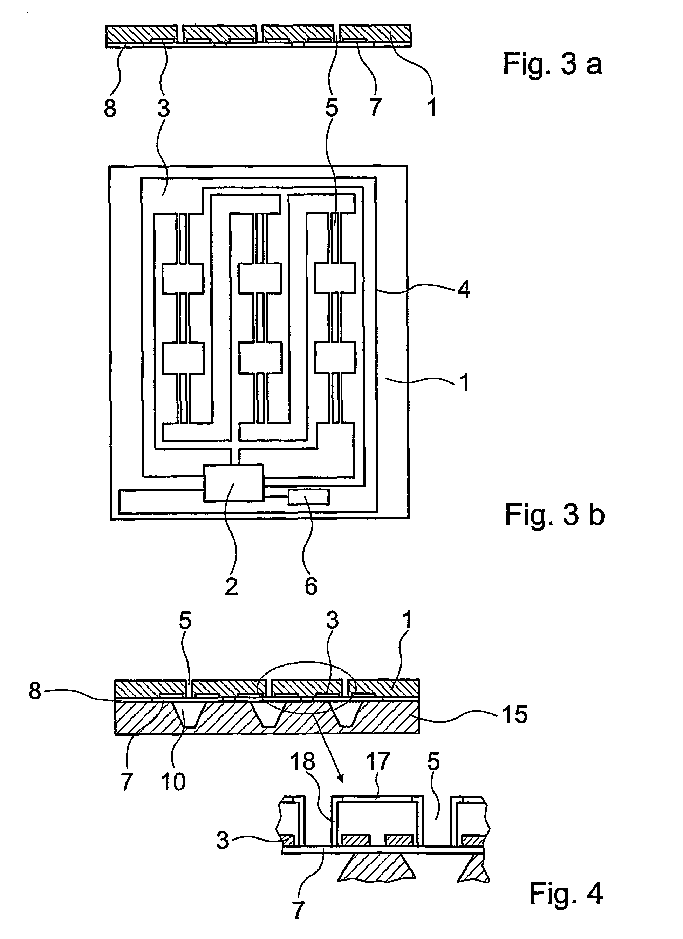

Zur Herstellung eines Folienmikrosystems wie dem der vorliegenden Erfindung wird in diesem Beispiel ein Siliziumwafer als Ausgangsmaterial eingesetzt. Auf dem Wafer werden einzelne Chips 1 mit integriertem Schaltkreis 2, Elektrodenstrukturen 3 zur Erzeugung der elektrischen Felder, isolierte Metallbahnen 4 zur Realisierung der Empfängerspule und Anschlusspads in Standardhalbleitertechnologie realisiert. Ein entsprechendes Layout ist beispielsweise in Fig. 3b zu erkennen. Nach dem Aufbringen dieser Strukturen wird der Wafer durch Ätzen der Rückseite auf etwa 10 µm gedünnt. Um eine hohe Zuverlässigkeit der elektronischen Schaltung zu gewährleisten, werden die Bereiche, in denen sich die aktiven Schaltkreise 2 befinden, lediglich auf 80 µm gedünnt.
Danach wird auf der Waferoberseite photolithographisch eine Ätzmaske hergestellt und durch reaktives Ionenätzen die Mikrospalte 5 erzeugt. Diese Mikrospalte 5 haben im vorliegenden Beispiel Dimensionen von 100 µm x 1 µm.
Aus Fig. 3b ist zu erkennen, dass die Elektroden 3 jeweils beidseitig der Mikrospalte 5 angeordnet sind. Die Mikrospalte 5 selbst sind arrayförmig in festen Abständen zueinander auf dem Träger 1 verteilt.

Als Energiespeicher werden im vorliegenden Beispiel SMD-Kondensatoren 6 durch Löten mit den dafür vorgesehenen Anschlussflächen auf den Chips 1 verbunden. Um die Biostabilität und Biokompatibilität der aufgebrachten Strukturen sicherzustellen, wird die Rückseite des Wafers, die mit der physiologischen Umgebung in direkten Kontakt kommt, durch Gasphasenabscheidung Parylene-beschichtet.

Das Aufbringen der elektroporösen Schicht 7 aus oxidiertem Cholesterol erfolgt nach dem Vereinzeln des Wafers zu einzelnen Chips. Die Beschichtung mit dem elektroporösen Material wird selektiv jeweils über den entsprechenden Mikrospalten 5 durchgeführt. Dazu wird der mit einer oder mehreren entsprechenden Öffnungen versehene Boden einer Schale zur Chipoberfläche justiert und an der entsprechenden Stelle, d.h. über den Mikrospalten, auf die Chipoberfläche gedrückt. In die Schale wird anschließend gelöstes oxidiertes Cholesterol gegeben. Das Lösungsmittel wird mit Hilfe eines Stickstoffstroms verdampft, so dass sich auf den durch die Öffnungen der Schale freiliegenden Bereichen der Chipoberfläche ein Film 7 aus oxidiertem Cholesterol bildet. Durch die geringe Öffnungsbreite der Mikrospalte werden diese vollständig durch diesen Film 7 bedeckt.
Das auf diese Weise entstandene flexible Mikrosystem kann anschließend mit der Oberseite, d.h. der Seite mit dem Film 7 aus oxidiertem Cholesterol, auf mit dem Wirkstoff gefüllte Reservoirs geklebt werden.

Fig. 3a zeigt ein auf diese Weise hergestelltes Mikrosystem in Schnittansicht. In dem Schnitt sind die Elektroden 3 beidseitig der Mikrokanäle 5, das Trägersubstrat 1, die Schicht 7 aus oxidiertem Cholesterol sowie ein Chipkleber 8 zur Befestigung des Mikrosystems auf Wirkstoffreservoirs zu erkennen. Durch die Ansteuerung des Films 7 aus oxidiertem Cholesterol über die jeweils beidseitig angeordneten Elektroden 3 lässt sich der Mikrospalt 5 für die Wirkstoffe durchgängig machen, so dass über diese Ansteuerung eine kontrollierte Wirkstofffreisetzung bewirkt werden kann. Sowohl die Triggersignale zur Ansteuerung des integrierten Schaltkreises 2 wie auch eine Energiezufuhr zum Aufladen des Kondensators 6 können über die integrierte Spule 4 von außerhalb des Körpers erfolgen, in den ein derartiges Mikrosystem eingebracht wird.

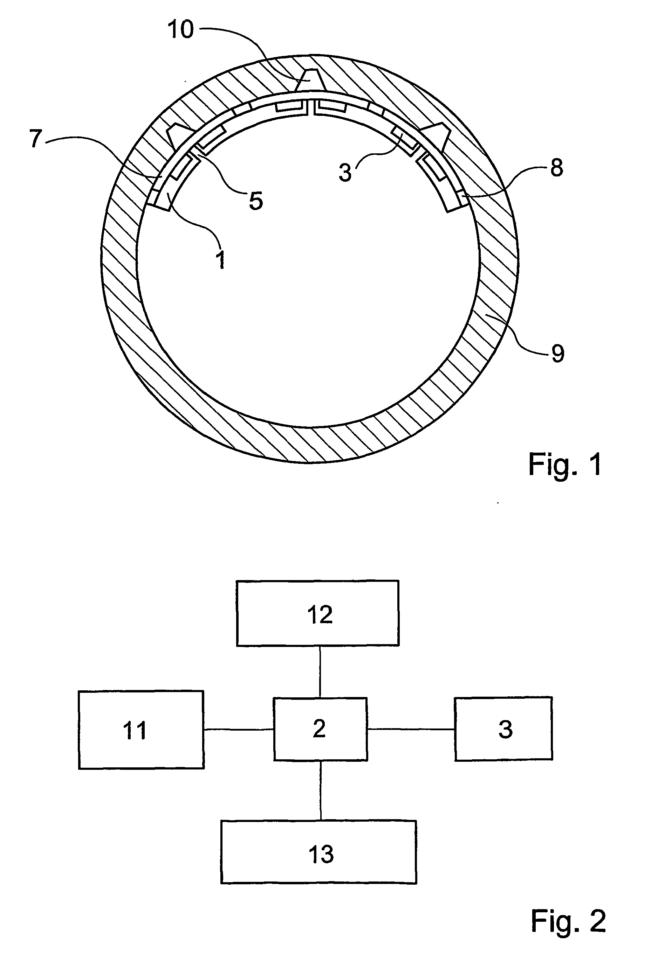

Fig. 1 zeigt eine schematische Schnittdarstellung eines flexiblen Folienmikrosystems wie dem der Fig. 3 zur kontrollierten Wirkstofffreisetzung in einer Prothese 9. In der Prothese 9 sind im vorliegenden Beispiel Wirkstoffreservoirs 10 eingearbeitet, in denen sich der Wirkstoff befindet. Das Mikrosystem wird in diesem Beispiel auf die Innenseite dieser Prothese 9 aufgeklebt, um auf diese Weise die Wirkstoffreservoirs über den Kleber 8 gegeneinander sowie gegen die Umgebung abzudichten. Durch Ansteuerung der elektroporösen Schicht 7 über die Elektroden 3 werden die Mikrospalte 5 für den Wirkstoff freigegeben, so dass dieser aus den Wirkstoffreservoirs 10 in die Umgebung abgegeben werden kann. Die in das flexible Trägersubstrat 1 integrierte Elektronik 2 ist in dieser Darstellung nicht zu erkennen.

Fig. 2 zeigt beispielhaft ein Blockschaltbild für die im vorliegenden Mikrosystem realisierbare Elektrodensteuerung. Die eigentliche Steuerung befindet sich im integrierten Schaltkreis 2. Über diesen werden die Elektroden 3 mit einer gewünschten Spannung bzw. einer Spannung mit einem bestimmten zeitlichen Verlauf beaufschlagt. Eine Triggerung des integrierten Schaltkreises 2 von außen kann über einen Biosensor, einen Programmspeicher oder eines Anschluss 11 für ein Triggersignal erfolgen. Weiterhin ist der integrierte Schaltkreis 2 mit einer Spannungsquelle 12 sowie einem Energiespeicher 13 bzw. einem Anschluss für den Energiespeicher verbunden. Die Energie bzw. Spannungsversorgung kann über eine entsprechende Empfängerspule realisiert werden, wie dies bereits vorangehend erläutert wurde.

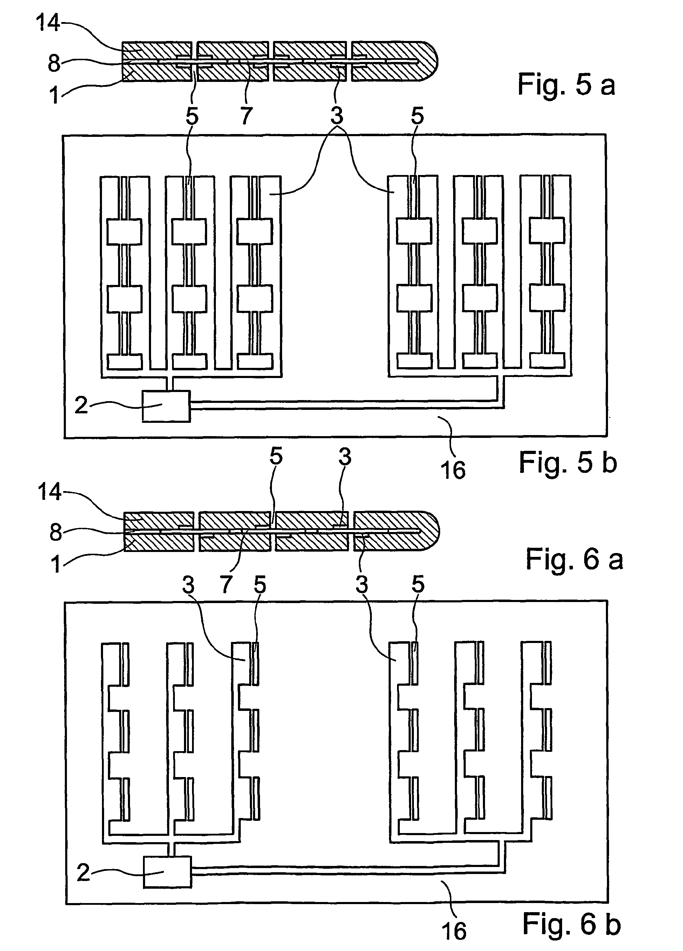

Fig. 4 zeigt ein Beispiel, bei ein Mikrosystem wie das der Fig. 3 auf einem Träger 15 mit Wirkstoffreservoirs aufgebracht ist. Die Verbindung zwischen dem Mikrosystem und dem Träger 15 kann beispielsweise über einen Chipkleber 8 erfolgen, der gleichzeitig die Abdichtung des Systems nach außen bewirkt.
In einem in der Figur gezeigten Ausschnitt dieses Mikrosystems sind ebenfalls Bereiche 17 an der Oberfläche des Trägersubstrates 1 zu erkennen, die ein Anhaften von Zellen begünstigen, sowie Bereiche 18, die das Anhaften gerade verhindern sollen. Durch diese Ausgestaltung der Oberfläche wird einerseits ein Einwachsen von Zellen in die Öffnungen 5 verhindert und so eine Schädigung der Lipidmembran vermieden und anderseits eine Fixierung des Systems im Körper durch Anwachsen der Zellen in den Bereichen 17 erreicht.

Die Figuren 5 bis 7 zeigen beispielhafte Ausführungsformen eines Mikrosystems, bei denen ein Lipidfilm 7 zwischen zwei Substraten 1, 14 eingeschlossen ist. Beide Substrate 1 und 14 sind in diesen Beispielen als Siliziumstrukuren ausgebildet. Durch diese Anordnungen wird zum einen der Lipidfilm 7 geschützt und zum anderen das Aufkleben des Mikrosystems auf Wirkstoffdepots wesentlich vereinfacht. Weiterhin können die Elektroden 3 beidseitig des Lipidfilms 7 realisiert werden, wodurch der elektrische Feldverlauf im Lipidfilm optimiert werden kann.

Zur Herstellung einer derartigen Struktur wird eine Siliziumstruktur mit einem Layout, wie sie in den Fig. 5b und 6b dargestellt ist, erzeugt. Fig. 5b zeigt hierbei ein Substrat 16, auf dem symmetrisch die Elektroden 3 aufgebracht sowie die Mikrospalte 5 eingeätzt sind. Weiterhin ist in dieser Layout-Ansicht die integrierte Elektronik 2 zu erkennen.
Die Herstellung einer derartigen Struktur kann mit einem Verfahren erfolgen, wie dies im Zusammenhang mit Fig. 3 bereits erläutert wurde. Nach der Herstellung dieser Struktur wird sie an der gestrichelt dargestellten Linie gefaltet, so dass die entsprechenden Mikrokanäle 5 übereinander zu liegen kommen. Hierdurch lässt sich eine Struktur mit beidseitig des Lipidfilms angeordneten Elektroden realisieren, wie sie in Fig. 5a in Schnittdarstellung zu erkennen ist. Die beiden Hälften des Substrates 16 werden über einen Chipkleber 8 zusammengehalten, nachdem eine Hälfte selektiv in der beschriebenen Weise mit einem Lipidfilm 7 beschichtet wurde.

Neben der in Fig. 5 gezeigten Anordnung mit vier Elektroden 3 im Bereich jedes Mikrospaltes 5 kann beispielsweise auch eine Anordnung realisiert werden, wie sie in Fig. 6 zu erkennen ist. Hier erfolgt die Herstellung des Mikrosystems in gleicher Weise wie bei Fig. 5 mit der Ausnahme, dass in jeder Hälfte des Substrates 16 jeweils nur eine Elektrode 3 an jedem Mikrokanal 5 angeordnet ist. Durch entsprechende Anordnung kann durch Falten dieses Substrates eine Struktur realisiert werden, bei der die beidseitig der Mikrokanäle angeordneten Elektroden jeweils wechselseitig am Lipidfilm 7 vorliegen (vgl. Fig. 6a). Durch die Anordnung der Elektroden kann ein gegenüber der Ausführungsform der Fig. 5 geänderter Feldverlauf im Lipidfilm 7 erzeugt werden.

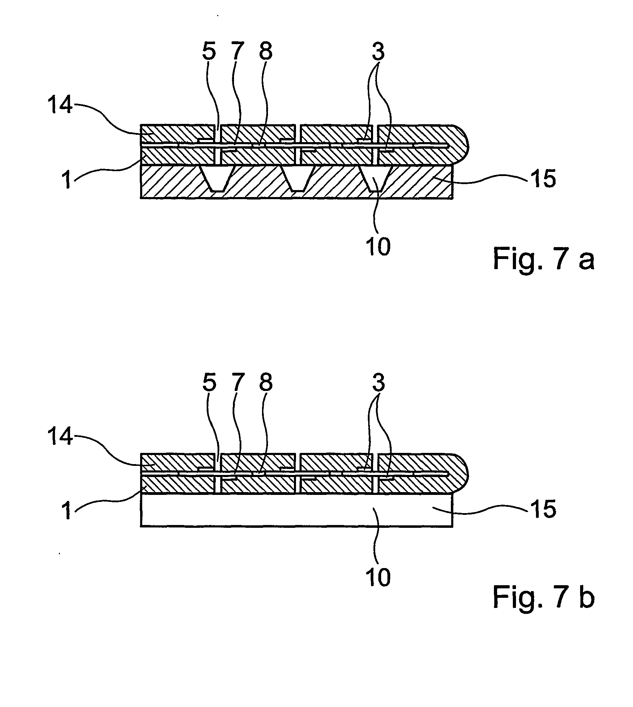

Fig. 7 zeigt schließlich eine Anordnung einer Struktur wie die der Fig. 5 oder 6 auf den entsprechenden Wirkstoffreservoirs 10. Bei der Ausführungsform der Fig. 7a ist das Mikrosystem auf einem Träger 15 mit Wirkstoffreservoirs 10 aufgeklebt. Die entsprechenden Mikrokanäle 5 befinden sich hierbei jeweils über den Wirkstoffreservoirs 10.
Das Wirkstoffdepot 10 kann auch als Polymerfilm ausgeführt sein, auf den das Mikrosystem ebenfalls vorzugsweise aufgeklebt wird. Der Polymerfilm enthält die abzugebenden Wirkstoffe und gibt diese mit einer definierten Rate durch die Mikrokanäle 5 ab (Fig. 7b).

Alternativ zu monolithisch integrierten Siliziumstrukturen, wie sie in den vorangegangenen Beispielen gezeigt wurden, kann das Foliensystem auch mit anderen flexiblen Trägermaterialien, beispielsweise Polyimid, realisiert werden. Die elektronische Steuerschaltung wird in diesem Fall separat als Siliziumschaltkreis hergestellt und in das Foliensystem hybrid integriert.

**BEZUGSZEICHENLISTE**

| | |
|---|---|
| 1 | Trägersubstrat bzw. Siliziumchip |
| 2 | Integrierte Elektronik |
| 3 | Elektroden |
| 4 | Empfängerspule |
| 5 | Mikrospalte bzw. Mikrokanäle |
| 6 | Kondensator |
| 7 | Elektroporöse Schicht bzw. Film |
| 8 | Chipkleber |
| 9 | Prothese |
| 10 | Wirkstoffreservoir |
| 11 | Anschluss für Triggersignal |
| 12 | Spannungsanschluss |
| 13 | Energiespeicher |
| 14 | Zweites Trägersubstrat |
| 15 | Träger mit Wirkstoffreservoirs |
| 16 | Substrat |
| 17 | Oberflächenbereich, der ein Anhaften von Zellen begünstigt |
| 18 | Oberflächenbereich, der ein Anhaften von Zellen verhindert |

## Patentansprüche

1. Mikrosystem zur Kontrolle der Wirkstoffabgabe aus einem Wirkstoffreservoir, mit
- einem dünnen Trägersubstrat (1), das aus einem für den Wirkstoff undurchlässigen Material besteht und
eine oder mehrere Durchgangsöffnungen (5) für den Wirkstoff aufweist,
- mehreren im Bereich der Durchgangsöffnungen (5) angeordneten Elektroden (3), und
- einer in das Trägersubstrat (1) integrierten Elektronik (2) zur Ansteuerung der Elektroden (3),
**dadurch gekennzeichnet,**
**dass** das Trägersubstrat (1) mechanisch flexibel ausgestaltet ist und die Durchgangsöffnungen (5) als Mikrokanäle und/oder Mikrospalte mit jeweils beidseitig angeordneten Elektroden (3) ausgebildet und auf einer Seite des Trägersubstrates (1) von einer Schicht (7) aus einem elektroporösen Material überdeckt sind.

2. Mikrosystem nach Anspruch 1,
**dadurch gekennzeichnet,**
**dass** die Mikrokanäle und/oder Mikrospalte (5) eine Öffnungsbreite von ≤ 10 µm aufweisen.

3. Mikrosystem nach einem der Ansprüche 1 oder 2,
**dadurch gekennzeichnet,**
**dass** die Schicht (7) aus elektroporösem Material zwischen dem Trägersubstrat (1) und einem weiteren Substrat (14) eingeschlossen ist, das in gleicher Weise wie das Trägersubstrat (1) mechanisch flexibel ausgestaltet und mit Mikrokanälen und/oder Mikrospalten (5) versehen ist, wobei die Mikrokanäle und/oder Mikrospalte (5) des weiteren Substrates (14) derart angeordnet sind, dass sie den Mikrokanälen bzw. Mikrospalten (5) des Trägersubstrates (1) gegenüber liegen.

4. Mikrosystem nach Anspruch 3,
**dadurch gekennzeichnet,**
**dass** die Anordnung aus Trägersubstrat (1) und weiterem Substrat (14) durch Faltung eines einzigen Substrates (16) gebildet ist.

5. Mikrosystem nach Anspruch 3 oder 4,
**dadurch gekennzeichnet,**
**dass** auf dem weiteren Substrat (14) jeweils beidseitig der Mikrokanäle und/oder Mikrospalte (5) Elektroden (3) angeordnet sind.

6. Mikrosystem nach einem der Ansprüche 1 bis 5,
**dadurch gekennzeichnet,**
**dass** die Elektroden (3) mit der Schicht (7) aus elektroporösem Material in Kontakt sind.

7. Mikrosystem nach einem der Ansprüche 1 bis 6,
**dadurch gekennzeichnet,**
**dass** die Elektroden (3) in das Trägersubstrat (1) und gegebenenfalls das weitere Substrat (14) integriert sind.

8. Mikrosystem nach einem der Ansprüche 1 bis 7,
**dadurch gekennzeichnet,**
**dass** die Schicht (7) aus elektroporösem Material zumindest Bereiche der Elektroden (7) überdeckt.

9. Mikrosystem nach einem der Ansprüche 1 bis 8,
**dadurch gekennzeichnet,**
**dass** die Mikrokanäle und/oder Mikrospalte (5) arrayförmig im Trägersubstrat (1) angeordnet sind.

10. Mikrosystem nach einem der Ansprüche 1 bis 9,
**dadurch gekennzeichnet,**
**dass** die Schicht (7) aus elektroporösem Material in Bereichen zwischen den Mikrokanälen und/oder Mikrospalten (5) auf dem Trägersubstrat (1) unterbrochen ist.

11. Mikrosystem nach einem der Ansprüche 1 bis 10,
**dadurch gekennzeichnet,**
**dass** Bereiche des Trägersubstrats (1) und gegebenenfalls des weiteren Substrats (14) mit biokompatiblem Material beschichtet sind.

12. Mikrosystem nach einem der Ansprüche 1 bis 11,
**dadurch gekennzeichnet,**
**dass** auf der Seite des Trägersubstrates (1), die der elektroporösen Schicht (7) gegenüber liegt, Bereiche (17) der Oberfläche zwischen den Mikrokanälen und/oder Mikrospalten (5) so ausgestaltet oder beschichtet sind, dass Zellen daran adhärieren können, und unmittelbar an die Mikrokanäle und/oder Mikrospalte (5) angrenzende Oberflächenbereiche (18) sowie die Innenwände der Mikrokanäle und/oder Mikrospalte (5) so ausgestaltet oder beschichtet sind, dass eine Zelladhäsion vermieden wird.

13. Mikrosystem nach einem der Ansprüche 1 bis 12,
**dadurch gekennzeichnet,**
**dass** das Trägersubstrat (1) und gegebenenfalls das weitere Substrat (14) durch ein gedünntes Si-Substrat gebildet werden.

14. Mikrosystem nach einem der Ansprüche 1 bis 12,
**dadurch gekennzeichnet,**
**dass** das Trägersubstrat (1) und gegebenenfalls das weitere Substrat (14) durch einen Polyimidfilm gebildet werden.

15. Mikrosystem nach einem der Ansprüche 1 bis 14,
**dadurch gekennzeichnet,**
**dass** das Trägersubstrat (1) und gegebenenfalls das weitere Substrat (14) eine Dicke von ≤ 80 µm aufweisen.

16. Mikrosystem nach einem der Ansprüche 1 bis 15,
**dadurch gekennzeichnet,**
**dass** ein Biosensor in das Trägersubstrat (1) integriert oder auf dieses aufgebracht ist, über den die Elektronik (2) getriggert wird.

17. Mikrosystem nach einem der Ansprüche 1 bis 15,
**dadurch gekennzeichnet,**
**dass** ein Programmspeicher in das Trägersubstrat (1) integriert oder auf dieses aufgebracht ist, über den die Elektronik (2) getriggert wird.

18. Mikrosystem nach einem der Ansprüche 1 bis 15,
**dadurch gekennzeichnet,**
**dass** eine Empfängerantenne (4) zum Empfang von Triggersignalen in das Trägersubstrat (1) integriert oder auf dieses aufgebracht und mit der Elektronik (2) verbunden ist.

19. Mikrosystem nach einem der Ansprüche 1 bis 18,
**dadurch gekennzeichnet,**
**dass** eine Empfängerspule (4) zur drahtlosen Spannungsversorgung in das Trägersubstrat (1) integriert oder auf dieses aufgebracht und mit der Elektronik (2) verbunden ist.

20. Mikrosystem nach einem der Ansprüche 1 bis 19,
**dadurch gekennzeichnet,**
**dass** ein Energiespeicher (6, 13) in das Trägersubstrat (1) integriert oder auf dieses aufgebracht und mit der Elektronik (2) verbunden ist.

21. Implantat mit einem Mikrosystem gemäß einem oder mehreren der vorangehenden Ansprüche mit Wirkstoffreservoirs (10), die an oder in einer Oberfläche des Implantats (9) fixiert sind und durch das am Implantat (9) befestigte Mikrosystem vollständig abgedeckt werden, wobei die Mikrospalten und/oder Mikrokanäle (5) jeweils über den Wirkstoffreservoirs (10) liegen.

22. Implantat nach Anspruch 21,
**dadurch gekennzeichnet,**
**dass** die Wirkstoffreservoirs als Vertiefungen in der Oberfläche des Implantats gebildet werden, die den Wirkstoff aufnehmen.

23. Implantat nach Anspruch 21,
**dadurch gekennzeichnet,**
**dass** die Wirkstoffreservoirs (10) durch eine auf die Oberfläche des Implantats (9) aufgebrachte Festphase des Wirkstoffes oder eines den Wirkstoff abgebenden Materials gebildet werden.

24. Implantat nach einem der Ansprüche 21 bis 23,
**dadurch gekennzeichnet,**
**dass** das Implantat (9) ein Herzkatheder oder ein Stent ist.

## Claims

1. A microsystem for controlling the active substance delivery from an active substance reservoir, having
- a thin carrier substrate (1), which comprises a material impermeable to the active substance and has one or more through openings (5) for the active substance,
- multiple electrodes (3) situated in the area of the through openings (5), and
- an electronic system (2), which is integrated into the carrier substrate (1), for activating the electrodes (3),
**characterized in that** the carrier substrate (1) is implemented as mechanically flexible and the through openings (5) are implemented as microchannels and/or microgaps having electrodes (3) situated on both sides in each case and are covered on one side of the carrier substrate (1) by a layer (7) made of an electroporous material.

2. The microsystem according to Claim 1,
**characterized in that** the microchannels and/or microgaps (5) have an opening width of ≤ 10 µm.

3. The microsystem according to one of Claims 1 or 2,
**characterized in that** the layer (7) made of electroporous material is enclosed between the carrier substrate (1) and a further substrate (14), which, in the same way as the carrier substrate (1), is implemented as mechanically flexible and is provided with microchannels and/or microgaps (5), the microchannels and/or microgaps (5) of the further substrate (14) being situated in such a way that they are diametrically opposite the microchannels or microgaps (5) of the carrier substrate (1).

4. The microsystem according to Claim 3,
**characterized in that** the system made of carrier substrate (1) and further substrate (14) is formed by folding a single substrate (16).

5. The microsystem according to Claim 3 or 4,
**characterized in that** electrodes (3) are situated on both sides of each of the microchannels and/or microgaps (5) on the further substrate (14).

6. The microsystem according to one of Claims 1 through 5,
**characterized in that** the electrodes (3) are in contact with the layer (7) made of electroporous material.

7. The microsystem according to one of Claims 1 through 6,
**characterized in that** the electrodes (3) are integrated in the carrier substrate (1) and if necessary the further substrate (14).

8. The microsystem according to one of Claims 1 through 7,
**characterized in that** the layer (7) made of electroporous material covers at least areas of the electrodes (7).

9. The microsystem according to one of Claims 1 through 8,
**characterized in that** the microchannels and/or microgaps (5) are situated in an array in the carrier substrate (1).

10. The microsystem according to one of Claims 1 through 9,
**characterized in that** the layer (7) made of electroporous material is interrupted in areas between the microchannels and/or microgaps (5) on the carrier substrate (1).

11. The microsystem according to one of Claims 1 through 10,
**characterized in that** areas of the carrier substrate (1) and if necessary the further substrate (14) are coated using biocompatible material.

12. The microsystem according to one of Claims 1 through 11,
**characterized in that** areas (17) of the surface between the microchannels and/or microgaps (5) are implemented or coated in such a way that cells may adhere thereon on the side of the carrier substrate (1) which is opposite the electroporous layer (7), and surface areas (18) directly adjoining the microchannels and/or microgaps (5) as well as the inner walls of the microchannels and/or microgaps (5) are implemented or coated in such a way that cell adhesion is avoided.

13. The microsystem according to one of Claims 1 through 12,
**characterized in that** the carrier substrate (1) and if necessary the further substrate (14) are formed by a thinned silicon substrate.

14. The microsystem according to one of Claims 1 through 12,
**characterized in that** the carrier substrate (1) and if necessary the further substrate (14) are formed by a polyimide film.

15. The microsystem according to one of Claims 1 through 14,
**characterized in that** the carrier substrate (1) and if necessary the further substrate (14) have a thickness of ≤ 80 µm.

16. The microsystem according to one of Claims 1 through 15,
**characterized in that** a biosensor is integrated in the carrier substrate (1) or applied thereto, via which the electronic system (2) is triggered.

17. The microsystem according to one of Claims 1 to 15,
**characterized in that** a program memory is integrated in the carrier substrate (1) or applied thereto, via which the electronic system (2) is triggered.

18. The microsystem according to one of Claims 1 through 15,
**characterized in that** a receiver antenna (4) for receiving trigger signals is integrated in the carrier substrate (1) or applied thereto and connected to the electronic system (2).

19. The microsystem according to one of Claims 1 through 18,
**characterized in that** a receiver coil (4) for wireless voltage supply is integrated in the carrier substrate (1) or applied thereto and connected to the electronic system (2).

20. The microsystem according to one of Claims 1 through 19,
**characterized in that** an energy accumulator (6, 13) is integrated in the carrier substrate (1) or applied thereto and connected to the electronic system (2).

21. An implant having a microsystem according to one more of the preceding claims having active substance reservoirs (10), which are fixed on or in a surface of the implant (9) and are completely covered by the microsystem attached to the implant (9), each of the microgaps and/or microchannels (5) lying over the active substance reservoirs (10).

22. The implant according to Claim 21,
**characterized in that** the active substance reservoirs are produced as depressions, which receive the active substance, in the surface of the implant.

23. The implant according to Claim 21,
**characterized in that** the active substance reservoirs (10) are formed by a solid phase of the active substance applied to the surface of the implant (9) or a material which delivers the active substance.

24. The implant according to one of Claims 21 through 23,
**characterized in that** the implant (9) is a cardiac catheter or a stent.

## Revendications

1. Micro-système pour le contrôle de la libération de substances actives à partir d'un réservoir, avec
- une fine couche de substrat (1) composée d'un matériau imperméable aux substances actives
et
une ou plusieurs ouvertures de passage (5) pour les substances actives,
- plusieurs électrodes (3) disposées dans la zone des ouvertures de passage (5), et
- une électronique (2) intégrée au substrat (1) pour la commande des électrodes (3),
**caractérisé en ce que**
le substrat (1) est mécaniquement flexible et les ouvertures de passage (5) sont conçues comme des micro-fentes et/ou micro-canaux avec des électrodes placées de chaque côté, et recouvertes d'une couche (7) de matière électroporeuse sur une des faces du substrat (1).

2. Micro-système selon la revendication 1,
**caractérisé en ce que**
les micro-fentes et/ou micro-canaux (5) ont une largeur d'ouverture de ≤ 10 µm.

3. Micro-système selon l'une des revendications 1 ou 2,
**caractérisé en ce que**
la couche électroporeuse (7) est enfermée entre la couche de substrat (1) et un deuxième substrat (14) aussi flexible que la base substrat (1) et pourvu de micro-fentes et/ou micro-canaux (5), les micro-fentes et/ou micro-canaux (5) du deuxième substrat (14) étant disposées de façon à se trouver en face des micro-fentes et/ou micro-canaux de la première couche de substrat (1).

4. Micro-système selon la revendication 3,
**caractérisé en ce que**
l'agencement d'un premier substrat (1) avec un deuxième substrat (14) est réalisé par pliage en deux d'un seul substrat (16).

5. Micro-système selon la revendication 3 ou 4
**caractérisé en ce que**
des électrodes (3) sont placées des deux côtés des micro-fentes et/ou micro-canaux (5) sur le deuxième substrat (14).

6. Micro-système selon l'une des revendications 1 à 5,
**caractérisé en ce que**
les électrodes (3) sont en contact avec la couche électroporeuse (7).

7. Micro-système selon l'une des revendication 1 à 6,
**caractérisé en ce que**
les électrodes (3) sont intégrées au substrat (1) et donc aussi dans le deuxième substrat (14).

8. Micro-système selon l'une des revendications 1 à 7,
**caractérisé en ce que**
la couche électroporeuse (7) recouvre au minimum certaines zones d'électrodes (7).

9. Micro-système selon l'une des revendications 1 à 8,
**caractérisé en ce que**
les micro-fentes et/ou micro-canaux (5) sont disposés en réseau dans la couche de substrat (1).

10. Micro-système selon l'une des revendications 1 à 9,
**caractérisé en ce que**
la couche électroporeuse (7) est interrompue dans les zones entre les micro-fentes et/ou micro-canaux (5) sur la première couche de substrat (1).

11. Micro-système selon l'une des revendications 1 à 10,
**caractérisé en ce que**
des zones du premier substrat (1) ou du deuxième substrat (14) sont recouvertes de matière biologiquement compatible.

12. Micro-système selon l'une des revendications 1 à 11,
**caractérisé en ce que**
sur la face de la base substrat (1) opposée à la couche électroporeuse (7), les zones (17) de surface entre les micro-fentes et/ou micro-canaux (5) sont conçues ou recouvertes de telle façon que des cellules peuvent y adhérer, tandis que les zones (18) de surface au niveau des ouvertures des micro-fentes et/ou micro-canaux (5) ainsi que les parois internes des micro-fentes et/ou micro-canaux sont conçues ou recouvertes de telle façon que l'adhésion des cellules est évitée.

13. Micro-système selon l'une des revendications 1 à 12,
**caractérisé en ce que**
le premier substrat (1) et selon les cas, le deuxième substrat (14), se composent de silicium dilué.

14. Micro-système selon l'une des revendications 1 à 12,
**caractérisé en ce que**
le premier substrat (1) et selon les cas, le deuxième substrat (14), se composent d'un film de polyimide.

15. Micro-système selon l'une des revendications 1 à 14,
**caractérisé en ce que**
le substrat (1) et selon les cas, le deuxième substrat, ont une épaisseur de s 80 µm.

16. Micro-système selon l'une des revendications 1 à 15,
**caractérisé en ce que**
un biosenseur est intégré dans ou placé sur le substrat (1), permettant d'activer l'électronique (2).

17. Micro-système selon l'une des revendications 1 à 15,
**caractérisé en ce que**
une mémoire de programme est intégrée dans le substrat (1) ou placée dessus, permettant d'activer l'électronique (2).

18. Micro-système selon l'une des revendications 1 à 15,
**caractérisé en ce que**
une antenne réceptrice (4) pour la réception des signaux de déclenchement est intégrée dans ou placée sur la couche de substrat (1) et reliée avec l'électronique (2).

19. Micro-système selon l'une des revendications 1 à 18,
**caractérisé en ce que**
une bobine réceptrice (4) est intégrée dans ou placé sur le substrat (1) et reliée avec l'électronique (2), pour permettre une alimentation en tension sans fil.

20. Micro-système selon l'une des revendications 1 à 19,
**caractérisé en ce que**
un accumulateur (6, 13) est intégré dans ou placé sur le substrat (1) et relié avec l'électronique.

21. Implant avec un micro-système selon une ou plusieurs des précédentes revendications, avec des réservoirs (10) de substances actives, qui sont fixés sur ou dans une des surfaces de l'implant (9) et entièrement recouverts par le micro-système monté sur l'implant (9), avec des micro-fentes et/ou micro-canaux (5) se superposant aux réservoirs à substances actives (10).

22. Implant selon la revendication 21,
**caractérisé en ce que**
les réservoirs à substances actives (10) sont formés dans la surface de l'implant (9) comme des renfoncements contenant les substances actives.

23. Implant selon la revendication 21,
**caractérisé en ce que**
les réservoirs à substances actives (10) se composent d'une phase solide de substances actives disposée sur la surface de l'implant (9) ou d'un matériau permettant de libérer les substances actives.

24. Implant selon l'une des revendications 21 à 23,
**caractérisé en ce que**
l'implant (9) consiste en un cathéter cardiaque ou une prothèse.
